# FASCICULE DE BREVET EUROPEEN

(11) **EP 0 975 314 B1**
(45) Date de publication et mention de la délivrance du brevet: **24.08.2005**
(21) Numéro de dépôt: 98963623.8
(22) Date de dépôt: 23.12.1998
(51) Int. Cl.: A61K 7/06

(54) **COMPOSITIONS COSMETIQUES CAPILLAIRES A BASE DE POLYMERES CATIONIQUES ET DE POLYMERES ASSOCIATIFS NON IONIQUES**
KOSMETISCHE ZUSAMMENSETZUNGEN ZUR HAARBEHANDLUNG AUF DER BASIS VON KATIONISCHEN POLYMEREN UND NICHTIONISHCEN ASSOZIATIVEN POLYMEREN
CAPILLARY COSMETIC COMPOSITIONS BASED ON CATIONIC POLYMERS AND NON-IONIC ASSOCIATIVE POLYMERS

(30) Priorité: 13.02.1998 FR 9801775
(43) Date de publication de la demande: 02.02.2000
(73) Titulaire: L'OREAL, 75008 Paris (FR)
(72) Inventeur: DOUIN, Véronique, F-75017 Paris (FR); LOPEZ, Juan, F-75020 Paris (FR); CERVANTES, Fréderic, F-75017 Paris (FR)
(74) Mandataire: Miszputen, Laurent
(86) Numéro de dépôt international: PCT/FR1998/002864
(87) Numéro de publication internationale: WO 1999/040891

(56) Documents cités:
- EP-A- 0 555 155
- EP-A- 0 824 914
- FR-A- 2 750 047
- US-A- 5 344 643
- US-A- 5 661 118

## Description

La présente invention concerne des compositions cosmétiques capillaires comprenant l'association d'au moins un polymère cationique et d'au moins un polymère associatif non ionique, ainsi que leur utilisation notamment en tant qu'après-shampooings.

L'importance des polymères cationiques en tant que composants cosmétiques pour le traitement et la protection des cheveux est connue depuis longtemps, Grâce à leur grande substantivité pour les cheveux, ils améliorent les propriétés cosmétiques de ceux-ci. Cependant, dans le domaine des soins capillaires, les polymères cationiques n'ont jamais pu remplacer avantageusement les tensioactifs cationiques de type ammonium quaternaire plus performants cosmétiquement mais qui présentent, pour la plupart d'entre eux, un certain nombre de problèmes tels qu'une tolérance oculaire imparfaite et une écotoxicité non négligeable.

Par ailleurs, pour l'épaississement d'après-shampooings, on fait classiquement appel à l'utilisation d'agents tensioactifs de type ammonium quaternaire en association avec des alcools gras. Ces émulsions épaisses présentent toutefois les inconvénients cumulés de leurs constituants, à savoir les problèmes décrits ci-dessus dû à la présence d'agents tensioactifs cationiques et un effet d'alourdissement des cheveux par dépôt des alcools gras.

L'utilisation d'épaississants classiques, c'est-à-dire de polymères hydrosolubles, n'a jusqu'ici pas permis d'obtenir des rhéologies satisfaisantes. Ces formulations sont souvent trop filantes et collantes dès lors que l'on atteint le niveau de viscosité recherché.

Une approche récente très intéressante a consisté à utiliser comme épaississants, des polymères capables de s'associer réversiblement entre eux ou avec d'autres molécules. De tels polymères sont appelés "polymères associatifs". Un cas particulier de polymères associatifs sont des polymères amphiphiles, c'est-à-dire des polymères comportant une ou plusieurs parties hydrophiles qui les rendent solubles dans l'eau, et une ou plusieurs zones hydrophobes par lesquelles les polymères interagissent et se rassemblent entre eux ou avec d'autres composants.

Il est connu de préparer des compositions capillaires sous forme de gel utilisant, comme système épaississant, de tels polymères amphiphiles associatifs, en conjonction avec des agents tensioactifs. On pense que les propriétés rhéologiques intéressantes des gels ainsi obtenus résultent de la formation de micelles mixtes formées par les agents tensioactifs et par les parties hydrophobes des polymères amphiphiles, ces micelles constituant une multitude de points de réticulation physique.

Le document EP 555 155 décrit une composition cosmétique comprenant au moins un tensioactif de type alkylpolyglycoside et/ou polyglycérolé et au moins un uréthannepolyéther, apte à être appliquée sur la peau ou les cheveux.

Le document FR 2 750 047 décrit une composition cosmétique ou dermatologique se présentant sous la forme d'un gel aqueux de forte viscosité, dont l'exemple 2 concerne un gel de coiffage comprenant un polymère anionique "RHEOVIS-CR®" de la Société Allied Colloids, en association avec le terpolymère de chlorure de diméthylammonium/acide acrylique/acrylamide vendu sous la dénomination MERQUAT PLUS® par la Société Merk.

Le document US 5 344 646 décrit une composition de conditionnement des cheveux contenant des agents de conditionnement, des tensioactifs, un polymère carboxyvinylique, un agent cationique et de l'eau.

Le document EP 824 914 décrit une composition épaississante pour le soin contenant, de manière générale, un polymère modificateur de rhéologie avec 0 à 30 % d'un polymère associatif.

La demande de brevet européen EP-A-0 415 705 décrit un système épaississant pour des après-shampooings à rincer constitué d'un premier agent épaississant qui est un un polymère hydrosoluble modifié par des groupes hydrophobes en C₈₋₂₂, et d'un épaississant secondaire qui est un polymère hydrosoluble ayant une masse molaire supérieure à environ 20 000.

La demanderesse a découvert à présent que la combinaison d'un certain type de polymère cationique ayant des propriétés cosmétiques intéressantes avec un polymère associatif non ionique permet non seulement d'obtenir des formulations épaissies d'une texture très agréable mais améliore également les performances cosmétiques du polymère cationique.

L'objet de la présente invention est par conséquent une composition cosmétique comprenant, dans un milieu cosmétiquement acceptable, au moins un polymère cationique contenant au moins un motif acrylamide ou dérivé d'acrylamide et au moins un polymère associatif non ionique contenant au moins une chaîne grasse.

Un autre objet de la présente invention est l'utilisation d'un polymère cationique contenant au moins un motif acrylamide ou dérivé d'acrylamide en association avec au moins un polymère associatif non ionique contenant au moins une chaîne grasse pour l'épaississement d'un milieu cosmétiquement acceptable.

Un troisième objet de l'invention est un procédé de traitement cosmétique des cheveux par les compositions cosmétiques épaissies par la combinaison d'un polymère cationique contenant au moins un motif acrylamide ou dérivé d'acrylamide et d'au moins un polymère associatif non ionique contenant au moins une chaîne grasse.

D'autres objets apparaîtront à la lecture de la description et des exemples qui suivront.

Les compositions cosmétiques conformes à l'invention sont indiquées à la revendication 1.

Le polymère cationique utilisé en tant que composant (A) est un copolymère réticulé de chlorure de méthacrylate de triméthylammonioéthyle et d'acrylamide, un exemple préféré étant commercialisé par la société ALLIED COLLOIDS sous la dénomination SALCARE® SC 92.

Conformément à l'invention on entend par "polymères associatifs" des polymères amphiphiles comportant des parties hydrophiles et des zones hydrophobes qui, en milieu aqueux, auront tendance à s'assembler entre eux ou avec d'autres molécules. Ces parties hydrophobes peuvent être par exemple des chaînes grasses latérales ou terminales introduites par copolymérisation ou par greffage, ou encore faire partie de la chaîne principale du polymère.

Les polymères amphiphiles non ioniques selon la présente invention utilisés comme composant (B) sont choisis de préférence parmi :
(1) les celluloses modifiées par des groupements comportant au moins une chaîne grasse en C₈₋₂₂, par exemple
   - l'hydroxyéthylcellulose modifiée par des groupements comportant au moins une chaîne grasse en C₈₋₂₂. Un tel produit est par exemple le NATROSOL PLUS GRADE 330 (chaînes alkyle en C₁₆) vendu par la société AQUALON ou encore le BERMOCOLL EHM 100 vendu par la société BEROL NOBEL; ou
   - les celluloses modifiées par des groupes polyalkylèneglycol éther d'alkylphénol, telles que le produit AMERCELL POLYMER® HM-1500 vendu par la société AMERCHOL;
(2) les hydroxypropylguars modifiés par des groupes comportant au moins une chaîne grasse en C₈₋₂₂ tels que le produit ESAFLOR® HM 22 commercialisé par la société LAMBERTI, et les produits MIRACARE® XC95-3 et RE205-1 vendus par la société RHONE POULENC;
(3) les polyuréthanes comportant au moins une chaîne grasse en C₈₋₂₂ comme les produits DAPRAL® T210 et DAPRAL® T212 vendus par la société AKZO, ou les produits ACRYSOL® 46 et DW 1206 F® commercialisés par la société ROHM & HAAS;
(4) les copolymères de vinylpyrrolidone et de monomères hydrophobes à chaîne grasse en C₈₋₂₂, comme par exemple les produits ANTARON® V216, ANTARON® V220, GANEX® V261 et GANEX® V220 commercialisés par la société I.S.P.;
(5) les copolymères de méthacrylates ou d'acrylates d'alkyle en C₁₋₆ et de monomères amphiphiles comportant au moins une chaîne grasse en C₈₋₂₂ tels que par exemple le copolymère méthacrylate de méthyle/acrylate de stéaryle oxyéthyléné vendu par la société GOLDSCHMIDT sous la dénomination ANTIL® 208;
(6) les copolymères de méthacrylates ou d'acrylates hydrophiles et de monomères hydrophobes comportant au moins une chaîne grasse en C₈₋₂₂ tels que par exemple le copolymère de méthacrylate de polyéthylèneglycol/méthacrylate de lauryle.
(7) les amidons à chaîne grasse,
(8) les protéines à chaîne grasse.

Les polymères cationiques et les polymères associatifs non ioniques de la présente invention sont utilisés dans les compositions de l'invention en des quantités suffisantes pour obtenir un épaississement satisfaisant du milieu aqueux permettant d'appliquer et de repartir la composition uniformément sur le cheveu. Une telle composition a une viscosité comprise entre 100 centipoises (cP) et 100 poises (P), de préférence entre 200 centipoises (cP) et 50 poises (P) .

On utilise notamment une quantité de polymère cationique comprise entre 0,05 et 10 % en poids, de préférence entre 0,1 et 5 % en poids, de matière active rapportée au poids total de la composition.

Les polymères associatifs non ioniques utilisés dans la présente invention, sont présents à raison de 0,05 à 10 % en poids, de préférence à raison de 0,1 à 5 % en poids, de matière active rapportée au poids total de la composition.

Le milieu cosmétiquement acceptable est constitué de préférence d'eau et peut contenir en outre des solvants cosmétiquement acceptables, par exemple des monoalcools inférieurs tels que l'éthanol ou l'isopropanol, des glycols tels que le diéthylèneglycol, des éthers de glycols tels que les alkyl-éthers d'éthylèneglycol ou de diéthylèneglycol, ou encore des esters d'acides gras, ces solvants étant utilisés seuls ou sous forme de mélange.

Les compositions cosmétiques capillaires de la présente invention peuvent contenir en outre un ou plusieurs additifs utilisés habituellement. On peut citer à titre d'exemple les alcools gras, esters d'alcools gras et d'acides gras, parfums, colorants, conservateurs, filtres solaires, protéines, protéines alkylées, protéines quaternisées, vitamines et provitamines, agents régulateurs de pH, agents tensioactifs anioniques, cationiques, non ioniques ou amphotères, silicones, silicones volatiles, huiles de silicone, gommes de silicone, silicones aminées, silicones quaternisées, silicones alkylées, silicones greffées, émulsions de silicone, huiles minérales et végétales, cires végétales, céramides ou pseudocéramides et des polymères autres que ceux selon l'invention définis ci-dessus. Il est bien entendu que le choix de ces composés doit tenir compte d'éventuelles interactions avec le système épaississant. L'homme du métier veillera à ce que l'adjonction de ces additifs n'ait pas d'influence défavorable sur les propriétés avantageuses des compositions obtenues selon la présente invention.

Un procédé de traitement cosmétique des cheveux selon l'invention consiste à appliquer une composition cosmétique définie ci-dessus sur les cheveux, à la répartir de façon homogène et, après un temps de pause approprié, à rincer éventuellement les cheveux ainsi traités avant de les sécher.

Les exemples suivants sont destinés à illustrer l'invention sans pour autant présenter un caractère limitatif.

### Exemples

Le Tableau 1 présente des compositions cosmétiques obtenues par association, dans de l'eau, de différents polymères cationiques (composant (A)) et d'un polymère associatif de la présente invention (composant (B)).
Les polymères cationiques présentés sont des copolymères d'acrylamide et de chlorure de méthacrylate de triméthylammonioéthyle réticulé (SALCARE® SC92 de la société ALLIED COLLOIDS) ou non réticulé (ROHAGIT® KF720 de la société ROHM)).
Les polymères de comparaison sont
- une hydroxyéthylcellulose réticulée à l'épichlorhydrine et portant des groupes triméthylammonium (CELQUAT® SC240, de Union Carbide,
- un copolymère de vinylpyrrolidone et de méthacrylate de diméthylaminoéthyle quaternisé (GAFQUAT® 855 de la société GAF), et
- un polycondensat de tétraméthylhexaméthylènediamine et de 1,3-dichloropropylène (Polymère C)
Il apparaît que seuls les polymères cationiques conformes à la présente invention donnent lieu à un épaississement plus important avec le polymère associatif en ayant leur effet cosmétique amélioré par ce dernier.

Le Tableau 2 présente des compositions cosmétiques associant, dans l'eau, un polymère cationique préféré de l'invention (composant (A)) à différents polymères associatifs conformes à l'invention (composant (B)). Tous ces polymères associatifs en conjonction avec le SALCARE® SC92 donnent un effet épaississant satisfaisant et permettent d'obtenir des compositions cosmétiques présentant d'excellentes performances cosmétiques.
On peut noter que ces résultats intéressants tant au niveau des propriétés cosmétiques que sur le plan de la formulation sont obtenus en absence d'agents tensioactifs cationiques.

Il est bien entendu que la description qui précède n'a été donnée qu'à titre purement illustratif et non limitatif et que des variantes ou des modifications peuvent y être apportées dans le cadre de la présente invention.

**Tableau 1**

| composant (A) | quantité de (A) (en % de matière active) | composant (B) | quantité de (B) en % de matière active | épaississement (viscosité en poises (P)) | propriétés cosmétiques |
|---|---|---|---|---|---|
| SALCARE SC92 | 0,5 | ACRYSOL 46 | 1 | très bon (viscosité : 18 P) | bonnes propriétés cosmétiques supérieures à celles de (A) seul |
| ROHAGIT KF720* | 0,95 | ACRYSOL 46 | 1 | bo (viscosité: 3,5 P)n | bonnes propriétés cosmétiques supérieures à |
| CELQUAT SC240 * | 0,5 | ACRYSOL 46 | 1 | pas d'épaississement | mauvaises propriétés cosmétiques identiques à celles de (A) seul |
| GAFQUAT 855 * | 0,5 | ACRYSOL 46 | 1 | pas d'épaississement | mauvaises propriétés cosmétiques identiques à celles de (A) seul |
| POLYMERE C * | 1 | ACRYSOL 46 | 1 | pas d'épaississement | identiques à celles de (A) seul |

| | | | | | |
|---|---|---|---|---|---|
| * comparatif | | | | | |

**Tableau 2**

| composant (A) | quantité de (A) (en % de matière active | composant (B) | quantité de (B) en % de matière active | épaississement viscosité en poises (P)) | propriétéss cosmétiques |
|---|---|---|---|---|---|
| SALCARE SC92 | 0,5 | ACRYSOL 46 | 1 | très bon viscosité: 18 P) | bonnes propriété cosmétiques supérieures à celles de (A) seul |
| SALCARE SC92 | 0,5 | DAPRAL T212 | 1 | bon viscosité: 4,9 P) | bonnes propriétés cosmétiques supérieures à celle de (A) seul |
| SALCARE SC92 | 0,5 | DW 1206 F | 1 | très bon (viscosité : 40 P) | bonnes propriétés cosmétiques supérieures à celles de (A) seul |
| SALCARE SC92 | 0,5 | NATROSOL PLUS | 1 | très bon (viscosité : 12 P) | bonnes propriétés cosmétiques supérieures celles de (A) seul |

## Revendications

1. Composition cosmétique **caractérisée par le fait qu'**elle comprend, dans un support cosmétiquement acceptable,
(A) au moins un polymère cationique contenant au moins un motif acrylamide ou dérivé d'acrylamide à savoir un copolymère réticulé de chlorure de méthacrylate de triméthylammonioéthyle et d'acrylamide,
(B) au moins un polymère associatif non ionique contenant au moins une chaîne grasse.

2. Composition cosmétique selon la revendication 1, **caractérisée par le fait que** les polymères associatifs non ioniques utilisés comme composant (B) sont choisis parmi :
(1) les celluloses modifiées par des groupements comportant au moins une chaîne grasse en C₈₋₂₂;
(2) les hydroxypropylguars modifiés par des groupes comportant au moins une chaîne grasse en C₈₋₂₂;
(3) les polyuréthannes comportant au moins une chaîne grasse en C₈₋₂₂;
(4) les copolymères de vinylpyrrolidone et de monomères hydrophobes à chaîne grasse en C₈₋₂₂;
(5) les copolymères de méthacrylates ou d'acrylates d'alkyle en C₁₋₆ et de monomères amphiphiles comportant au moins une chaîne grasse;
(6) les copolymères de méthacrylates ou d'acrylates hydrophiles et de monomères hydrophobes comportant au moins une chaîne grasse en C₈₋₂₂;
(7) les amidons à chaîne grasse; et
(8) les protéines à chaîne grasse.

3. Composition cosmétique selon l'une quelconque des revendications 1 à 2, **caractérisée par le fait qu'**elle contient le composant (A) à raison de 0,05 à 10 % en poids, de préférence à raison de 0,1 à 5 % en poids, de matière active rapportée au poids total de la composition.

4. Composition cosmétique selon l'une quelconque des revendications 1 à 3, **caractérisée par le fait qu'**elle contient le composant (B) à raison de 0,05 à 10 % en poids; de préférence à raison de 0,1 à 5 % en poids, de matière active rapportée au poids total de la composition.

5. Composition cosmétique selon l'une quelconque des revendications 1 à 4, **caractérisée par le fait que** le support cosmétiquement acceptable est constitué d'eau.

6. Composition cosmétique selon la revendication 5, **caractérisée par le fait que** le support cosmétiquement acceptable contient, en outre, des solvants cosmétiquement acceptables choisis parmi les monoalcools inférieurs tels que l'éthanol ou l'isopropanol, les glycols tels que le diéthylèneglycol, les éthers de glycols tels que les alkyléthers d'éthylèneglycol ou de diéthylèneglycol, ou les esters d'acides gras, ces solvants étant utilisés seuls ou sous forme de mélange.

7. Composition cosmétique selon l'une quelconque des revendications 1 à 6, **caractérisée en ce qu'**elle contient, en outre, un ou plusieurs additifs utilisés habituellement, choisis parmi les alcools gras, esters d'alcools gras et d'acides gras, parfums, colorants, conservateurs, filtres solaires, protéines, protéines alkylées, protéines quaternisées, vitamines et provitamines, agents régulateurs de pH, agents tensioactifs anioniques, cationiques, non ioniques ou amphotères, silicones, silicones volatiles, huiles de silicone, gommes de silicone, silicones aminées, silicones quaternisées, silicones alkylées, silicones greffées, émulsions de silicone, huiles minérales et végétales, cires végétales, céramides ou pseudocéramides et des polymères autres que ceux définis dans l'une quelconque des revendications 1 à 3.

8. Composition cosmétique selon l'une quelconque des revendications 1 à 7, **caractérisée par le fait qu'**elle se présente sous forme d'un après-shampooing.

9. Utilisation d'un polymère cationique comportant au moins un motif acrylamide ou dérivé d'acrylamide en association avec au moins un polymère non ionique associatif contenant au moins une chaîne grasse en tant que système épaississant d'une composition cosmétique, le polymère cationique étant un copolymère réticulé de chlorure de méthacrylate de triméthylammonioéthyle et d'acrylamide.

10. Procédé de traitement cosmétique des cheveux, **caractérisé par le fait qu'**on met en contact les cheveux avec une composition cosmétique définie selon l'une quelconque des revendications 1 à 8 et que l'on rince éventuellement les cheveux ainsi traités, après un temps de pause donné, avant de les sécher.

## Patentansprüche

1. Kosmetische Zusammensetzung, **dadurch gekennzeichnet, dass** sie in einem kosmetisch akzeptablen Träger enthält:
(A) mindestens ein kationisches Polymer, das mindestens eine Acrylamideinheit oder eine von Acrylamid abgeleitete Einheit enthält, nämlich ein vernetztes Copolymer aus dem Chlorid von Trimethylammonioethylmethacrylat und Acrylamid, und
(B) mindestens ein nichtionisches assoziatives Polymer, das mindestens eine Fettkette enthält.

2. Kosmetische Zusammensetzung nach Anspruch 1, **dadurch gekennzeichnet, dass** die als Komponente (B) verwendeten nichtionischen assoziativen Polymere ausgewählt sind unter:
(1) Cellulosen, die mit Gruppen modifiziert sind, die mindestens eine C₈₋₂₂-Fettkette aufweisen;
(2) Hydroxypropylguargummen, die mit Gruppen modifiziert sind, die mindestens eine C₈₋₂₂-Fettkette aufweisen;
(3) Polyurethanen, die mindestens eine C₈₋₂₂-Fettkette enthalten;
(4) Copolymeren von Vinylpyrrolidon und hydrophoben Monomeren mit C₈₋₂₂-Fettkette;
(5) Copolymeren von C₁₋₆-Alkylmethacrylaten oder C₁₋₆-Alkylacrylaten und amphiphilen Monomeren, die mindestens eine Fettkette enthalten;
(6) Copolymeren von hydrophilen Methacrylaten oder Acrylaten und hydrophoben Monomeren, die mindestens eine C₈₋₂₂-Fettkette enthalten;
(7) Stärkeverbindungen mit Fettkette; und
(8) Proteinen mit Fettkette.

3. Kosmetische Zusammensetzung nach einem der Ansprüche 1 oder 2, **dadurch gekennzeichnet, dass** sie die Komponente (A) in einer Menge von 0,05 bis 10 Gew.-% und vorzugsweise in einer Menge von 0,1 bis 5 Gew.-% wirksame Substanz, bezogen auf das Gesamtgewicht der Zusammensetzung, enthält.

4. Kosmetische Zusammensetzung nach einem der Ansprüche 1 bis 3, **dadurch gekennzeichnet, dass** sie die Komponente (B) in einer Menge von 0,05 bis 10 Gew.-% und vorzugsweise in einer Menge von 0,1 bis 5 Gew.-% wirksame Substanz, bezogen auf das Gesamtgewicht der Zusammensetzung, enthält.

5. Kosmetische Zusammensetzung nach einem der Ansprüche 1 bis 4, **dadurch gekennzeichnet, dass** der kosmetisch akzeptable Träger aus Wasser besteht.

6. Kosmetische Zusammensetzung nach Anspruch 5, **dadurch gekennzeichnet, dass** der kosmetisch akzeptable Träger ferner kosmetisch akzeptable Lösungsmittel enthält, die unter den niederen Monoalkoholen, wie Ethanol oder Isopropanol, Glykolen, wie Diethylenglykol, Glykolethern, wie Ethylenglykolalkylethern oder Diethylenglykolalkylethern, oder Estern von Fettsäuren ausgewählt sind, wobei die Lösungsmittel einzeln oder als Gemisch verwendet werden.

7. Kosmetische Zusammensetzung nach einem der Ansprüche 1 bis 6, **dadurch gekennzeichnet, dass** sie ferner einen oder mehrere gewöhnlich verwendete Zusatzstoffe enthält, die unter den Fettalkoholen, Estern von Fettalkoholen und Fettsäuren, Parfums, Farbmitteln, Konservierungsmitteln, Sonnenschutzfiltern, Proteinen, alkylierten Proteinen, quaternisierten Proteinen, Vitaminen, Provitaminen, pH-Reglern, anionischen, kationischen, nichtionischen oder amphoteren grenzflächenaktiven Stoffen, Siliconen, flüchtigen Siliconen, Siliconölen, Silicongummis, aminierten Siliconen, quaternisierten Siliconen, alkylierten Siliconen, gepfropften Siliconen, Siliconemulsionen, Mineralölen, pflanzlichen Ölen, pflanzlichen Wachsen, Ceramiden, Pseudoceramiden und Polymeren, die von den in einem der Ansprüche 1 bis 3 definierten Polymeren verschieden sind, ausgewählt sind.

8. Kosmetische Zusammensetzung nach einem der Ansprüche 1 bis 7, **dadurch gekennzeichnet, dass** sie in Form eines nach der Haarwäsche aufzutragenden Produkts vorliegt.

9. Verwendung eines kationischen Polymers, das mindestens eine Acrylamideinheit oder eine von Acrylamid abgeleitete Einheit aufweist, in Kombination mit mindestens einem assoziativen nichtionischen Polymer, das mindestens eine Fettkette enthält, als Verdickungssystem für eine kosmetische Zusammensetzung, wobei das kationische Polymer ein vernetztes Copolymer des Chlorids von Trimethylammonioethylmethacrylat und Acrylamid ist.

10. Verfahren zur kosmetischen Behandlung der Haare, **dadurch gekennzeichnet, dass** die Haare mit einer Zusammensetzung nach einem der Ansprüche 1 bis 8 in Kontakt gebracht werden und **dadurch**, dass die behandelten Haare nach einer gegebenen Einwirkzeit gegebenenfalls gespült werden, bevor sie getrocknet werden.

## Claims

1. Cosmetic composition, **characterized in that** it comprises, in a cosmetically acceptable support,
(A) at least one cationic polymer containing at least one acrylamide, or acrylamide derivative, unit, namely a crosslinked trimethylammonioethyl methacrylate chloride-acrylamide copolymer,
(B) at least one nonionic associative polymer containing at least one fatty chain.

2. Cosmetic composition according to Claim 1, **characterized in that** the nonionic associative polymers used as component (B) are chosen from:
(1) celluloses modified with groups comprising at least one C₈-C₂₂ fatty chain;
(2) hydroxypropyl guars modified with groups comprising at least one C₈-C₂₂ fatty chain;
(3) polyurethanes comprising at least one C₈-C₂₂ fatty chain;
(4) copolymers of vinylpyrrolidone and hydrophobic monomers containing a C₈-C₂₂ fatty chain;
(5) copolymers of C₁-C₆ alkyl methacrylates or acrylates and amphiphilic monomers comprising at least one fatty chain;
(6) copolymers of hydrophilic methacrylates or acrylates and hydrophobic monomers comprising at least one C₈-C₂₂ fatty chain;
(7) fatty-chain starches; and
(8) fatty-chain proteins.

3. Cosmetic composition according to either of Claims 1 and 2, **characterized in that** it contains the component (A) in a proportion of from 0.05 to 10% by weight, preferably in a proportion of from 0.1 to 5% by weight, of active material relative to the total weight of the composition.

4. Cosmetic composition according to any one of Claims 1 to 3, **characterized in that** it contains the component (B) in a proportion of from 0.05 to 10% by weight, preferably in a proportion of from 0.1 to 5% by weight, of active material relative to the total weight of the composition.

5. Cosmetic composition according to any one of Claims 1 to 4, **characterized in that** the cosmetically acceptable support consists of water.

6. Cosmetic composition according to Claim 5, **characterized in that** the cosmetically acceptable support also contains cosmetically acceptable solvents chosen from lower monoalcohols such as ethanol or isopropanol, glycols such as diethylene glycol, glycol ethers such as ethylene glycol alkyl ether or diethylene glycol alkyl ether, or fatty acid esters, these solvents being used alone or in the form of a mixture.

7. Cosmetic composition according to any one of Claims 1 to 6, **characterized in that** it also contains one or more commonly used additives chosen from fatty alcohols, esters of fatty alcohols and of fatty acids, fragrances, dyes, preserving agents, sunscreens, proteins, alkylated proteins, quaternized proteins, vitamins and provitamins, pH regulators, anionic, cationic, nonionic or amphoteric surfactants, silicones, volatile silicones, silicone oils, silicone gums, amino silicones, quaternized silicones, alkylated silicones, grafted silicones, silicone emulsions, mineral and plant oils, plant waxes, ceramides or pseudoceramides and polymers other than those defined in any one of Claims 1 to 3.

8. Cosmetic composition according to any one of Claims 1 to 7, **characterized in that** it is in the form of a conditioner.

9. Use of a cationic polymer comprising at least one acrylamide unit or acrylamide derivative in combination with at least one nonionic associative polymer containing at least one fatty chain, as a thickening system for a cosmetic composition, the cationic polymer being a crosslinked trimethylammonioethyl methacrylate chloride-acrylamide copolymer.

10. Cosmetic process for treating hair, **characterized in that** a cosmetic composition defined according to any one of Claims 1 to 8 is placed in contact with the hair and, after leaving it on the hair for a given period, the hair thus treated is optionally rinsed and is then dried.
